# EUROPEAN PATENT SPECIFICATION

(11) **EP 4 527 359 B1**
(45) Date of publication and mention of the grant of the patent: **25.11.2026**
(21) Application number: 25156595.8
(22) Date of filing: 04.12.2019
(51) Int. Cl.: A61F 2/95

(54) **DELIVERY SYSTEMS FOR PROSTHETIC HEART VALVE**
FREISETZUNGSSYSTEME FÜR HERZKLAPPENPROTHESE
SYSTÈMES DE POSE POUR VALVULE CARDIAQUE PROTHÉTIQUE

(30) Priority: 11.12.2018 US 201862778159 P
(43) Date of publication of application: 26.03.2025
(62) Document number(s) of the earlier application(s) in accordance with Art. 76 EPC: 24189956.6 / 4 461 270; 19828091.9 / 3 893 817
(73) Proprietor: Edwards Lifesciences Corporation, Irvine, CA 92614 (US)
(72) Inventor: MURAD, Michael C., Irvine, CA 92614 (US); BIALAS, Michael R., Irvine, CA 92614 (US)
(74) Representative: Eisenführ Speiser

(56) References cited:
- US-A- 5 951 569
- US-A1- 2013 030 519

## Description

### CROSS-REFERENCE TO RELATED APPLICATIONS

This application claims the benefit of U.S. Provisional Application 62/778,159 filed on December 11, 2018.

### FIELD

The present disclosure concerns embodiments of delivery systems for implanting prosthetic heart valves.

### BACKGROUND

Prosthetic cardiac valves have been used for many years to treat cardiac valvular disorders. The native heart valves (such as the aortic, pulmonary and mitral valves) serve critical functions in assuring the forward flow of an adequate supply of blood through the cardiovascular system. These heart valves can be rendered less effective by congenital, inflammatory or infectious conditions. Such damage to the valves can result in serious cardiovascular compromise or death. For many years, the definitive treatment for such disorders was the surgical repair or replacement of the valve during open heart surgery, but such surgeries are prone to many complications. Because of the drawbacks associated with conventional open-heart surgery, percutaneous and minimally-invasive surgical approaches are garnering intense attention. In one technique, a prosthetic valve is configured to be implanted in a much less invasive procedure by way of catheterization.

In this technique, a prosthetic valve is mounted in a crimped state on the end portion of a flexible catheter and advanced through a blood vessel of the patient until the prosthetic valve reaches the implantation site. The prosthetic valve at the catheter tip is then expanded to its functional size at the site of the defective native valve such as by inflating a balloon on which the prosthetic valve is mounted. For instance, U.S. Patent Nos. 5,411,522 and 6,730,118 describe collapsible transcatheter heart valves that can be percutaneously introduced in a compressed state on a catheter and expanded in the desired position by balloon inflation or by utilization of a self-expanding frame or stent.

A typical delivery apparatus for a transcatheter heart valve has a radiopaque marker that can be viewed under fluoroscopy. During delivery of the prosthetic valve, a physician can use the radiopaque marker to align the prosthetic valve with the implantation site. Using a conventional delivery apparatus, tension exerted on portions of the apparatus can result in the radiopaque marker being shifted relative to the prosthetic valve which can result in deployment of the prosthetic valve at the incorrect location.

Accordingly, a need exists for improved devices and methods for accurately determining a location of a prosthetic valve during an implantation procedure.

US 2013/0030519 A1 (US'519) describes systems and methods for delivering prosthetic devices, such as prosthetic heart valves, through the body and into the heart for implantation therein. The prosthetic devices delivered with the delivery systems described in US' 519 are, for example, radially expandable from a radially compressed state mounted on the delivery system to a radially expanded state for implantation using an inflatable balloon of the delivery system. Exemplary delivery routes through the body and into the heart include transfemoral routes, transapical routes, and transaortic routes, among others.

US 5,951,569 (US'569) provides a stent delivery system comprising an inflation shaft having an expandable balloon sealingly mounted at the distal end thereof and a tubular sheath having the inflation shaft longitudinally running therethrough. The tubular sheath has an expandable sheath at the distal end thereof with the balloon longitudinally running therethrough and an expandable stent mounted on the expandable sheath. US'569 also provides a method of delivering a stent into a body lumen. The method comprises the steps of providing an inflation shaft having an expandable balloon sealingly mounted at the distal end thereof and providing a tubular sheath having the inflation shaft longitudinally running therethrough. The expandable sheath has the balloon longitudinally and slidingly running therethrough, and an expandable stent is mounted on the expandable sheath. The stent is positioned in the body lumen at the treatment site and the balloon is inflated simultaneously with the expandable sheath to deploy the stent.

### SUMMARY

The claimed invention is defined in independent claim 1 and relates to a delivery assembly comprising, inter alia, a balloon-expandable prosthetic heart valve, and a delivery apparatus. Some preferred configurations of the claimed invention are defined in the dependent claims. Also described herein are related aspects, examples, embodiments and arrangements useful for understanding the claimed invention, and which do not necessarily constitute embodiments of the claimed invention. Features recited in the independent claim without any indication of being optional are to be understood as obligatory, irrespective whether or not conditional language is used in the following description. The subject-matter for which protection is sought is defined by the claims.

Described herein are embodiments of a delivery assembly and method for implanting a prosthetic heart valve. The assembly can be used to precisely position a prosthetic valve at an implantation site within a patient.

In one representative embodiment, a delivery apparatus can include a handle, a first shaft extending from the handle, and a second shaft extending coaxially over the first shaft. The first and second shafts can have proximal end portions coupled to the handle and distal end portions. The delivery apparatus can further include a shoulder coupled to the distal end portion of the second shaft. The shoulder can have a flared end portion configured to abut a prosthetic valve positioned around the first shaft in a radially compressed state. The shoulder can further include an extension portion extending distally from the shoulder and at least one radiopaque marker disposed on the extension portion at a location within the prosthetic valve when the prosthetic valve is in a radially compressed configuration on the first shaft.

In some embodiments, the extension portion can be a tubular member, for example, a tubular member extending coaxially over the first shaft.

In some embodiments, the delivery apparatus further comprises a balloon disposed over the first shaft. In some embodiments, the shoulder can comprise an annular wall proximal to the flared portion. The annular wall can comprise one or more radially extending openings that allow an inflation fluid inside of the shoulder to flow outwardly through the one or more openings into the balloon.

In some embodiments, the shoulder can be a first shoulder and the delivery apparatus can further comprise a second shoulder coupled to the distal end portion of the first shaft. The first and second shoulders can be disposed within the balloon.

In some embodiments, the extension portion extends axially from a first location adjacent the first shoulder to a second location axially between the first and second shoulders. The second location can be about equidistant between the first and second shoulders.

In some embodiments, the at least one radiopaque marker comprises gold, platinum, radiopaque nitinol, and combinations thereof.

The delivery assembly defined in claim 1 includes a balloon-expandable prosthetic heart valve radially expandable between a crimped configuration and an expanded configuration and a delivery apparatus. The delivery apparatus comprises a handle, a first shaft extending from the handle, a second shaft extending from the handle coaxially over the first shaft, and a balloon. The first and second shafts have proximal end portions coupled to the handle and distal end portions. The balloon is disposed over the first shaft. The delivery apparatus further comprises a proximal shoulder and a distal shoulder extending coaxially over the first shaft and disposed within the balloon. The proximal shoulder includes an extension portion extending from the proximal shoulder and at least one radiopaque marker on the extension portion. The prosthetic heart valve is disposed on the balloon in the radially crimped configuration between the proximal and distal shoulders such that the at least one radiopaque marker is positioned within the prosthetic heart valve.

In some embodiments, the proximal shoulder can be coupled to a distal end portion of the second shaft and the distal shoulder can be coupled to a distal end portion of the first shaft.

In some embodiments, the at least one radiopaque marker can comprise gold, platinum, radiopaque nitinol, and combinations thereof.

In some embodiments, the first shaft can extend coaxially through the extension portion and is not secured directly to the extension portion. The extension portion can have a proximal end connected to the proximal shoulder and a distal end location axially between the proximal shoulder and the distal shoulder.

In another representative embodiment, a method of implanting a prosthetic heart valve, can comprise inserting a distal end portion of a delivery apparatus into the body of a patient. A balloon-expandable prosthetic heart valve can be coupled to the distal end portion of the delivery apparatus in a radially crimped configuration. The delivery apparatus can comprise a handle, a first shaft extending from the handle, and a second shaft extending from the handle over the first shaft. The delivery apparatus can further comprise a proximal shoulder extending over the first shaft, a distal shoulder extending over the first shaft, and a balloon disposed over the proximal and distal shoulders and the inner shaft. The proximal shoulder can include an extension portion extending from the proximal shoulder coaxially over the inner shaft having at least one radiopaque marker on the extension portion located within the prosthetic valve. The method can further include advancing the distal end portion of the delivery apparatus and the prosthetic valve distally through the vasculature of the patient to an implantation site, aligning the radiopaque marker with respect to the implantation site using fluoroscopy, and deploying the prosthetic valve to an expanded configuration at the implantation site.

In some embodiments, the implantation site can be a native aortic valve annulus of the patient's heart.

In some embodiments, the act of aligning the radiopaque marker with respect to the implantation site can comprise aligning the radiopaque marker with the native valve annulus.

The various innovations of this disclosure can be used in combination or separately. This summary is provided to introduce a selection of concepts in a simplified form that a further described below in the detailed description. This summary is not intended to identify key features or essential features of the claimed subject matter. The foregoing and other objects, features, and advantages of the disclosure will become more apparent from the following detailed description, which proceeds with reference to the accompanying figures.

### BRIEF DESCRIPTION OF THE DRAWINGS

FIG. 1 is a perspective view of a representative embodiment of a prosthetic heart valve.
FIG. 2 is a side elevation view of a representative embodiment of a delivery apparatus for implanting a prosthetic heart valve.
FIG. 3 is side elevation view of a distal end portion of the delivery apparatus of FIG. 2.
FIG. 4 is a cross-sectional view of a portion of the delivery apparatus of FIG. 2.
FIG. 5 is a partial cross-sectional view of the delivery apparatus of FIG. 2.

### DETAILED DESCRIPTION

Described herein are systems and methods for delivering prosthetic devices, such as prosthetic heart valves, through the body and into the heart for implantation therein.

Prosthetic valves disclosed herein can be radially compressible and expandable between a radially compressed configuration and a radially expanded configuration. Thus, the prosthetic valves can be crimped on an implant delivery apparatus in the radially compressed configuration during delivery, and then expanded to the radially expanded configuration once the prosthetic valve reaches the implantation site.

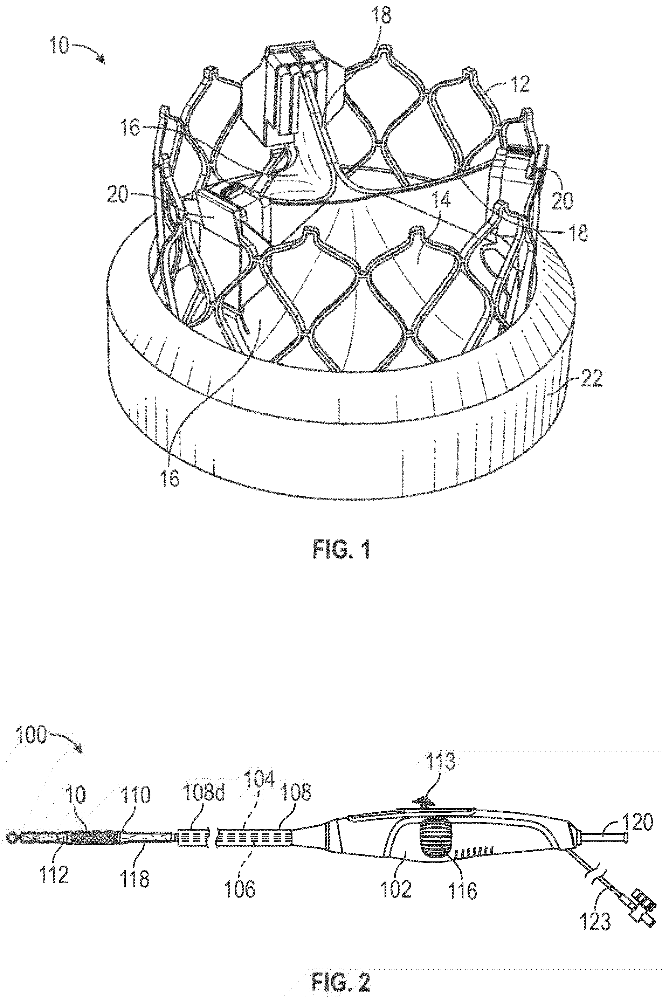

FIG. 1 shows an exemplary prosthetic valve 10, according to one embodiment. In particular embodiments, the prosthetic valve 10 can be implanted within the native aortic annulus, although it also can be implanted at other locations in the heart, including within the native mitral valve, the native pulmonary valve, and the native tricuspid valve. Prosthetic heart valve 10 comprises a stent or frame 12 and a valvular structure 14 supported by the frame and configured to regulate the flow of blood through the prosthetic valve.

In some embodiments, the prosthetic valve 10 is adapted to be implanted in the native aortic valve and can be implanted in the body using a delivery apparatus (see e.g., delivery apparatus 100 described below). The frame 12 can comprise a plastically expandable material, such as stainless steel, a nickel based alloy (e.g., a nickel-cobalt-chromium alloy), polymers, or combinations thereof, so that the frame 12 plastically expands when the prosthetic valve expands from the crimped, radially compressed configuration (FIG. 2) to the radially expanded configuration (FIG. 1) upon actuation of the delivery apparatus, for example, by inflating a balloon.

The valvular structure 14 can comprise plurality of leaflets 16 mounted inside of the frame. The opposing sides of each leaflet 16 can be paired with adjacent leaflets to form commissures 18 of the valvular structure. The commissures 18 can be mounted to the frame via reinforcing members 20. The prosthetic valve 10 can also include a sealing member 22 mounted on the outside of the frame. The sealing member 22 is configured to help seal the prosthetic valve against surrounding tissue and prevent or minimize perivalvular leakage. The leaflets 16 can be made from any of various suitable biocompatible materials, including natural tissue, such as bovine pericardial tissue (or pericardial tissue from other sources) or synthetic materials, such as any of various fabrics or non-fabric materials (e.g., polyurethane). The reinforcing members 20 and the sealing member 22 desirably are made of a fabric material, such as polyethylene terephthalate (PET) fabric, although non-fabric materials and natural tissue also could be used. Further details of the prosthetic valve 10 are disclosed in U.S. Patent Application Publication No. 2018/0028310. Other types of prosthetic heart valves that can deployed using any of the devices and methods disclosed herein are described in U.S. Patent Nos. 7,510,575; 7,993,394; and 9,393,110.

FIG. 2 shows a delivery apparatus 100, according to one embodiment, adapted to deliver a prosthetic heart valve, such as the illustrated prosthetic heart valve 10. The prosthetic heart valve 10 can be, for example, a prosthetic aortic valve that is adapted to be implanted in the native aortic valve, although in other embodiments the prosthetic valve 10 can be implanted in any of the other native valves of the heart (the native mitral, tricuspid, or pulmonary valves).

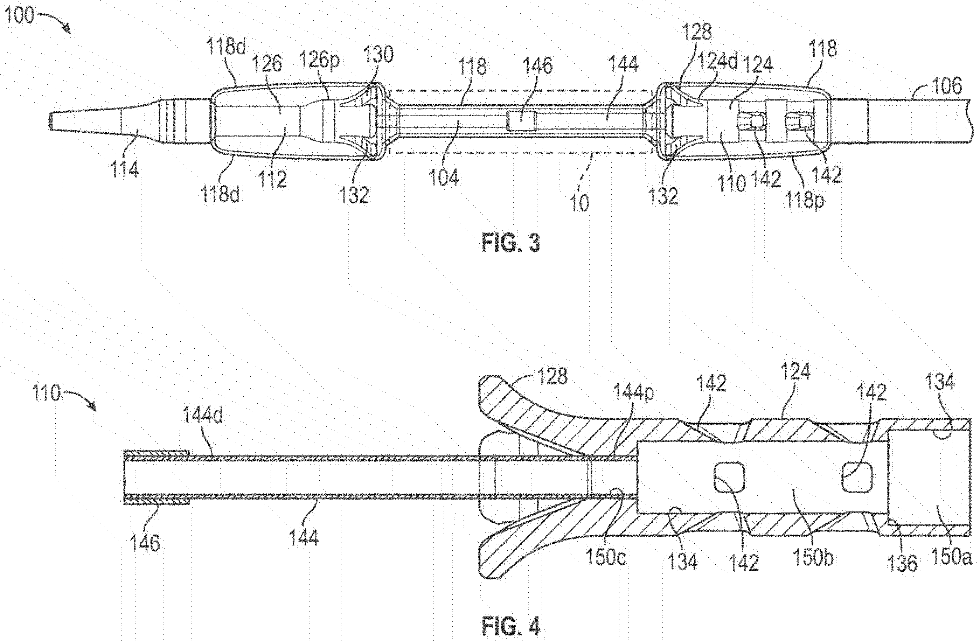

The delivery apparatus 100 in the illustrated embodiment generally includes a handle 102, a first elongated shaft 104 extending distally from the handle 102, and a second shaft 106 extending co-axially over the first shaft 104, a third shaft 108 extending co-axially over the second shaft 106, one or more shoulders (e.g., a proximal shoulder 110 and a distal shoulder 112), and a nose cone 114 (see FIG. 3). In other embodiments, the delivery apparatus can include only a first shoulder (e.g., a proximal shoulder).

The first shaft 104 is the inner-most shaft in the illustrated embodiment and can be referred to as the inner shaft of the delivery apparatus 100. The second shaft 106 is the middle shaft in the illustrated embodiment and can be referred to as the intermediate shaft 106. Likewise, the third shaft 108 is the outer-most shaft 108 in the illustrated embodiment and can be referred to as the outer shaft or outer sheath of the delivery apparatus 100. The shafts 104, 106, 108 can be axially and/or rotationally movable relative to each other to facilitate delivery and positioning of the prosthetic valve 10 at an implantation site in the patient's body. The handle 102 can include one or more adjustment mechanisms configured to produce relative movement between the shafts. For example, the handle 102 can include a slideable adjustment lever 113 that is operatively connected to the second shaft 106. Thus, axial motion of the lever 113 can produce axial movement of the second shaft 106 (and, hence, of the prosthetic valve 10) in the proximal and distal directions relative to the first shaft 104.

The nose cone 114 (FIG. 3) can be connected to a distal end of the inner shaft 104. A guide wire (not shown) can extend through a central lumen of the inner shaft 104 and an inner lumen of nose cone 114 so that the delivery apparatus 100 can be advanced over the guide wire inside the patient's vasculature. The outer shaft 108 can also include a steerable portion 108d, the curvature of which can be adjusted by the operator to assist in guiding the apparatus through the patient's vasculature and, in particular, the aortic arch. The steerable portion 108d can be coupled to a control device (e.g., knob 116) by at least one pull wire, such that tensioning and releasing the pull wire causes corresponding flexing and unflexing of the steerable section 108d of the outer shaft 108 and therefore of the first and second shafts 104, 106. Further details regarding the steering of the delivery apparatus are described in U.S. Patent Application Publication 2017/0065415.

The proximal ends of the shafts 104, 106, 108 can be coupled to the handle 102. During delivery of a prosthetic valve, the handle 102 can be maneuvered by a physician to advance or retract the delivery apparatus through the patient's vasculature. In some embodiments, the handle 102 can include one or more adjustment mechanisms, for example, knob 116 and lever 113, or other actuating mechanisms for controlling different components of the delivery apparatus 100 in order to expand and/or deploy the prosthetic valve.

The shafts 104, 106, 108 can be formed from any of various suitable materials and techniques, such as any of various polymers, including nylon, or a polyether block amide (commercially available as Pebax^{®}), which can be extruded to form the shafts. In other examples, any of the shafts 104, 106, 108 can comprise a braided layer formed from metal (e.g., Nitinol or stainless steel) or polymeric wires, or a combination of a one or more braided layers and one or more extruded polymeric layers. The shafts can have longitudinal sections formed from different materials in order to vary the flexibility of the shafts along their lengths. The first shaft 104 can have an inner liner or layer formed of a low friction material, such as polytetrafluoroethylene (commercially available as Teflon^{®}) to minimize sliding friction with a guide wire.

An inflatable balloon 118 can be mounted along the distal end portions of the first and second shafts 104, 106 and can extend over the proximal and distal shoulders 110, 112. As best shown in FIG. 3, the balloon 118 has a proximal end portion 118p that can be secured to the outer surface of the second shaft 106 and a distal end portion 118d that can be secured to the outer surface of the nose cone 114 or the outer surface of the distal shoulder 112. As depicted in FIG. 2, the prosthetic valve 10 can be radially crimped between the shoulders 110, 112 over the balloon 118 for delivery into a patient's vasculature. Once the prosthetic valve 100 reaches the desired implantation site (e.g., within the native aortic valve), the balloon 118 can be inflated to radially expand the prosthetic valve against the surrounding tissue, as described in more detail below.

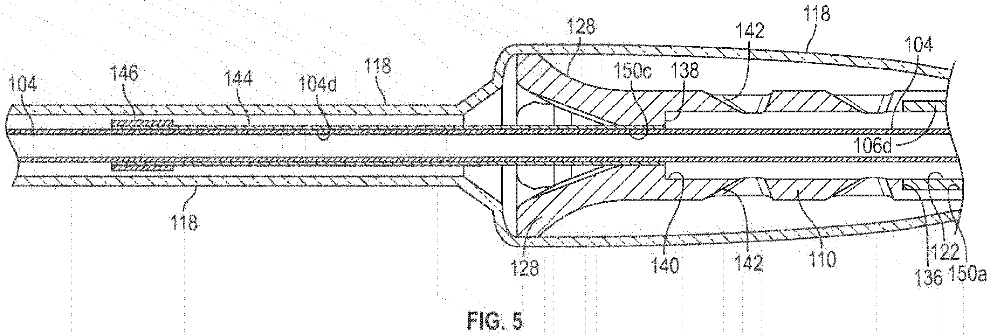

The delivery apparatus 100 can include a proximal port 120 extending from the handle 102. The first and second shafts 104, 106 can be sized such that a first annular space 122 (see FIG. 5) is defined between the first and second shafts 104, 106 along the entire length of the second shaft 106. In use, the proximal port 120 can be fluidly connected to a fluid conduit (e.g., tubing), which in turn can be fluidly connected to a fluid source (e.g., a syringe containing an inflation fluid, such as saline) to inflate the balloon 118. Thus, the fluid conduit can be in fluid communication with the first annular space 122 between the first and second shafts 104, 106 such that fluid can flow through the delivery apparatus and inflate the balloon 118, as described in more detail below. The handle 102 can further include a side arm 123 which can be, for example, a flush tube having an internal passage that fluidly communicates with the first annular space 122.

Further details regarding the delivery apparatus 100 and methods for delivering and deploying a prosthetic valve using the delivery apparatus can be found, for example, in U.S. Publication No. 2017/0065415. Other examples of delivery apparatuses that can be used to implant a prosthetic heart valve with devices disclosed herein are described in U.S. Patent Publication No. 2009/0281619.

Referring now to FIG. 3, as noted above, the delivery apparatus comprises one or more shoulders. In the illustrated embodiment, the delivery apparatus 100 includes a proximal shoulder 110 and a distal shoulder 112.

The distal shoulder 112 can be connected to a distal end portion of the first or inner shaft 104 and/or the nose cone 114. In some embodiments, the nose cone 114 and the distal shoulder 112 can be integrally formed as a single component. In other embodiments, the nose cone 114 and the distal shoulder 112 can be separately formed and connected to each other during the process of assembling the delivery apparatus. In other embodiments, the nose cone 114 and the distal shoulder 112 can be spaced apart from each other along the first shaft 104. The proximal shoulder 110 can be connected to a distal end portion 106d (FIG. 5) of the second shaft 106. The proximal and distal shoulders 110, 112 can be disposed within the balloon 118.

Each shoulder 110, 112 can comprise a body 124, 126, respectively. In some embodiments, each shoulder can further comprise a flared end portion 128, 130, respectively. The flared end portion 130 of the distal shoulder 112 can be located at a proximal end portion 126p of the body 126, and the flared end portion 128 of the proximal shoulder 110 can be located at a distal end portion 124d of the body 124. Each flared end portion 128, 130 can have a diameter equal to or slightly greater than a diameter of the prosthetic valve 10 when in the compressed or crimped configuration on the balloon 118. The flared end portions 128, 130 of the shoulders 110, 112 can define a space sized to at least partially receive the prosthetic valve 10 when it is crimped on the balloon 118. In this way, the flared end portions 128, 130 help restrain the prosthetic valve 10 against movement relative to the delivery apparatus 100 during the implantation procedure.

The flared end portions 128, 130 can each comprise one or more slots 132. In the illustrated embodiment, each flared end portion comprises four slots 132 that are equally spaced circumferentially about the flared end portion. However, other embodiments can comprise a greater or fewer number of slots. The slots 132 facilitate radial compression of the flared end portions 128, 130, which is advantageous during manufacturing of the delivery apparatus and during crimping of the prosthetic valve 10. The flared end portions 128, 130 can be radially compressed to a smaller diameter using slots 132 for insertion into the balloon 118 and then allowed to expand once inside the balloon 118. In addition, the slots 132 can facilitate the flow of fluid through the balloon 118, as discussed in more detail below. Further details regarding the shoulders of the delivery apparatus and methods for delivering and deploying a prosthetic valve using the delivery apparatus can be found, for example, in U.S. Publication Nos. 2013/0030519 and 2017/0065415.

Referring now to FIG. 4, the body 124 of the proximal shoulder 110 can have an annular shape defining an inner lumen. The inner lumen of the proximal shoulder 110 can be formed with a stepped surface 134 extending through the proximal shoulder 110. The stepped surface 134 can be formed with a first annular lip 136 and a second annular lip 138, so as to define a proximal lumen portion 150a, an intermediate lumen portion 150b, and a distal lumen portion 150c.

Referring now to FIG. 5, the distal end portion 106d of the second shaft 106 extends partially into the proximal lumen portion 150a and abuts the first annular lip 136. The distal end portion 106d of the second shaft 106 can be fixedly secured within the proximal shoulder 110, such as with an adhesive, welding, and/or a friction or interference fit. The distal lumen portion 150c can be sized such that the first shaft 104 can extend therethrough. The intermediate lumen portion 150b and the first shaft 104 can be sized such that a second annular space 140 is defined radially between the outer surface of the first shaft 104 and the inner surface of the body 124 and axially between the first annular lip 136 and the second annular lip 138. The second annular space 140 is in fluid communication with the first annular space 122 such that an inflation fluid can flow through the first annular space 122 into the second annular space 140.

The proximal shoulder 110 can comprise one or more openings 142 formed in the annular wall between the outer and inner surfaces of the proximal shoulder 110. The openings 142 can be in fluid communication with the second annular space 140 such that an inflation fluid can flow from the second annular space 140 outwardly through the openings 142 and into the balloon 118. For example, an inflation fluid from the fluid source can flow through the fluid conduit, through the first annular space 122, through the second annular space 140, outwardly through the openings 142, and into the balloon 118 to inflate the balloon and deploy the prosthetic valve 10. The slots 132 in the flared end portions 128, 130 of the shoulders 110, 112 facilitate the flow of inflation fluid distally through the flared end portion 128 of the proximal shoulder, through the region of the balloon 118 extending through the prosthetic valve 10, through the flared end portion 130 of the distal shoulder 112, and into a distal region of the balloon 118d (FIG. 3), thus facilitating even expansion of the balloon 118.

Referring again to FIG. 4, the proximal shoulder 110 comprises an extension portion 144. The extension portion 144 can extend distally from the body 124 of the proximal shoulder 110. The extension portion 144 can be, for example, a tubular member or shaft extending over a distal end portion 104d of the first shaft 104 (FIG. 5). The extension portion 144 can have a proximal end portion 144p that extends into the distal lumen portion 150c of the proximal shoulder and is fixedly secured therein.

In the illustrated embodiment, the extension portion 144 has a cylindrical shape and a circular cross-sectional profile in a plane perpendicular to the longitudinal axis of the delivery apparatus. In other examples, the extension portion can have any of various other shapes. For example, the extension portion 144 can have a cross-sectional shape in a plane perpendicular to the longitudinal axis of the delivery apparatus that does not extend completely around the first shaft 104, such as a C-shape. In another implementation, the extension portion can be a relatively flat component, such as beam-like component having a rectangular or square shaped cross-sectional profile, and can extend along one side of the first shaft 104.

In some embodiments, the extension portion 144 can extend from the proximal shoulder 110 at least a length equal to half the length of the prosthetic valve 10 when in the compressed configuration. The extension portion 144 can be formed from any of various suitable materials, for example, the extension portion 144 can be formed from a polymer (nylon or Pebax) such that chafing or scoring between extension portion 144 and the first shaft 104 is mitigated.

In the illustrated embodiment, the extension portion 144 is fixedly secured to the proximal shoulder 110 by overmolding the proximal shoulder 110 onto an end portion, for example the proximal end portion 144p, of the extension portion 144. In other embodiments, the end portion 144p can be fixedly secured within the distal lumen portion 150c of the proximal shoulder 110 using, for example, an adhesive, welding, and/or an interference fit. In other embodiments, the extension portion 144 can be fixedly secured to the proximal shoulder without extending into the distal lumen portion 150c.

In alternative embodiments, the extension portion 144 can be integrally formed as part of the proximal shoulder 110, meaning that the extension portion 144 and the proximal shoulder have a one-piece, unitary construction without any fasteners, welds, or adhesives securing the extension portion 144 to the proximal shoulder 110. For example, the proximal shoulder 110 can be formed by a molding process (e.g., injection molding) wherein the extension portion 144 is formed as part of the proximal shoulder 110 during the molding process.

The extension portion 144 comprises at least one radiopaque marker 146. The radiopaque marker 146 can be disposed on the extension portion 144 at a location within the prosthetic valve 10 when the prosthetic valve 10 is in a radially compressed configuration between the proximal and distal shoulders 110, 112. For example, the radiopaque marker 146 can be disposed on, or extend from a distal end portion 144d of the extension portion 144.

In a conventional delivery apparatus, the radiopaque marker typically is disposed on the innermost shaft extending through the balloon. As a result, any movement of the innermost shaft relative to the prosthetic valve, such as may be caused by compression or deflection of the innermost shaft as the delivery apparatus is steered around the aortic arch, can displace the radiopaque marker from its intended position within the prosthetic valve, which can inhibit precise alignment of the prosthetic valve at the intended deployment location.

However, in the illustrated embodiment, the radiopaque marker 146 has a fixed position relative to the proximal shoulder 110 by virtue of the extension portion 144. The extension portion 144 desirably is not fixed to the distal end portion 104d of the first shaft and therefore the distal end portion 104d is able to move relative to the proximal shoulder 110 if the distal end portion undergoes compression or deflection. Any movement of the distal end portion 104d of the first shaft 104 therefore does not result in movement of the marker 146 relative to the proximal shoulder 110 and the prosthetic valve 10. Therefore, by placing the radiopaque marker 146 on the extension portion 144, the physician can position the prosthetic valve 10 at a selected implantation location within the patient, for example, the native aortic annulus with much greater precision and accuracy compared with known delivery apparatuses.

In the illustrated embodiment, the radiopaque marker 146 is a marker band extending annularly around a distal end portion 144d of the extension portion 144. In other embodiments, the radiopaque marker can be a series of radiopaque bands or a radiopaque pattern and can be disposed along the length of the extension portion 144 (e.g., spaced apart from each other along the length of the extension portion 144)). Also, the radiopaque markers need not be annular bands that extend completely around the extension portion 144. Instead, the radiopaque markers can comprise any of various shapes, such as partial bands, or axially extending lines. In still other embodiments, the radiopaque marker can extend from the distal end portion 144d of the extension portion 144. The radiopaque marker 146 can comprise, for example, gold, platinum, radiopaque nitinol, and combinations thereof.

In other embodiments, in lieu of or in addition to the radiopaque marker 146, the extension portion 144 itself can comprise a radiopaque material such as gold, platinum, radiopaque nitinol, or combinations thereof. Some or all of the extension portion 144 can be formed from radiopaque material. In such embodiments, the distal end of the extension portion 144 can be used to position the prosthetic valve 10 at the selected implantation location within the patient.

The delivery apparatus 100 can be used to deliver and implant a prosthetic heart valve, for example, prosthetic heart valve 10, in the following exemplary manner. The prosthetic valve 10 can be crimped on the distal end portion of delivery apparatus 100, between the proximal and distal shoulders 110, 112 such that extension portion 144 comprising marker band 146 is disposed within the prosthetic valve 10. The distal end of delivery apparatus 100 (along with prosthetic valve 10) can be advanced through the vasculature of a patient to a selected implantation site (e.g., the native aortic annulus). A physician can use the radiopaque marker 146 to align the prosthetic valve with the implantation site, for example, by visualizing the marker 146 using fluoroscopy.

Once the physician has determined that the prosthetic valve 10 is positioned as desired at the implantation site, the prosthetic valve 10 can be deployed (e.g., radially expanded). To deploy the prosthetic valve, an inflation fluid from a source (e.g., a syringe) is introduced under pressure into the first annular space 122 through the length of the second shaft 106, through the second annular space 140, out the openings 142, and into the balloon 118, thus inflating the balloon 118 and expanding the prosthetic valve 10 so as to contact the native annulus. Other examples of methods for implanting a prosthetic valve can be found, for example, in U.S. Publication No. 2017/0065415.

As mentioned above, the length of the extension portion 144 in the illustrated embodiment is selected such that the marker 146 is at the middle or mid-section of the prosthetic valve 10 (halfway between the inflow and outflow ends of the prosthetic valve) during delivery. In this manner, the marker 146 can be used to align the mid-section of the prosthetic valve with a particular location or area of the patient's anatomy. In other embodiments, the extension portion 144 can have different lengths and the marker 146 can be at another location within the prosthetic valve, such as at the inflow end or the outflow end of the prosthetic valve, to enable alignment of another section of the prosthetic valve with a particular location or area of the patient's anatomy. In one specific implementation, the extension portion 144 can extend the entire length between the proximal and distal shoulders 110, 112 and one or more markers 146 can be positioned at selected locations along the length of the extension portion 144, such as at locations corresponding to the inflow end, mid-section, and outflow end of the prosthetic valve.

As noted above, the stationary position of the radiopaque marker 146 with respect to the proximal shoulder 110 prevents the marker from being shifted from its intended location relative to the prosthetic valve 10, thus allowing the prosthetic valve 10 to be precisely positioned within the native annulus without interference caused by the movement of the first shaft 104 relative to the proximal shoulder 110.

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

All features described herein are independent of one another and, except where structurally impossible, can be used in combination with any other feature described herein.

As used in this application and in the claims, the singular forms "a," "an," and "the" include the plural forms unless the context clearly dictates otherwise. Additionally, the term "includes" means "comprises." Further, the terms "coupled" and "associated" generally mean electrically, electromagnetically, and/or physically (e.g., mechanically or chemically) coupled or linked and does not exclude the presence of intermediate elements between the coupled or associated items absent specific contrary language.

In the context of the present application, the terms "lower" and "upper" are used interchangeably with the terms "inflow" and "outflow", respectively. Thus, for example, the lower end of the valve is its inflow end and the upper end of the valve is its outflow end.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device toward the user, while distal motion of the device is motion of the device away from the user. The terms "longitudinal" and "axial" refer to an axis extending in the proximal and distal directions, unless otherwise expressly defined.

## Claims

1. A delivery assembly, comprising:
a balloon-expandable prosthetic heart valve (10) radially expandable between a crimped configuration and an expanded configuration; and
a delivery apparatus (100) comprising:
a handle (102),
a first shaft (104) extending from the handle (102) and having a proximal end portion coupled to the handle (102) and a distal end portion,
a second shaft (106) extending coaxially over the first shaft (104) and having a proximal end portion coupled to the handle (102) and a distal end portion;
a balloon (118) disposed over the first shaft (104);
a proximal shoulder (110) and a distal shoulder (112) extending coaxially over the first shaft (104) and disposed within the balloon (118);
an extension portion (144) extending from the proximal shoulder (110);
at least one radiopaque marker (146) on the extension portion (144); and
wherein the prosthetic heart valve (10) is disposed on the balloon (118) in the radially crimped configuration between the proximal and distal shoulders (112) such that the at least one radiopaque marker (146) is positioned within the prosthetic heart valve (10).

2. The assembly of claim 1, wherein the distal shoulder (112) is coupled to the distal end portion of the first shaft (104).

3. The assembly of any one of the preceding claims, wherein the first shaft (104) extends coaxially through the extension portion (144) and is not secured directly to the extension portion (144).

4. The assembly of any one of the preceding claims, further comprising a nose cone (114) connected to the distal end portion of the first shaft (104), preferably wherein a guidewire can extend through a central lumen of the first shaft (104) and an inner lumen of the nose cone (114) so that the delivery apparatus (100) can be advanced over the guide wire inside the patient's vasculature.

5. The assembly of claim 4, wherein the balloon (118) has a proximal end portion secured to an outer surface of the second shaft (106) and a distal end portion secured to an outer surface of the nose cone (114) or an outer surface of the distal shoulder (112).

6. The assembly of any one of claims 4 or 5, wherein the nose cone (114) and the distal shoulder (112) are spaced apart from each other along the first shaft (104).

7. The assembly of any one of the preceding claims, wherein the extension portion (144) is a tubular member or shaft extending over the distal end portion of the first shaft (104).

8. The assembly of claim 7, wherein the extension portion (144) has a proximal end portion that extends into a distal lumen portion of the proximal shoulder (110) and is fixedly secured therein.

9. The assembly of any one of the preceding claims, wherein the extension portion (144) is not fixed to the distal end portion of the first shaft (104).

10. The assembly of any one of the preceding claims, wherein the radiopaque marker (146) is a series of radiopaque bands or a radiopaque pattern and is disposed along the length of the extension portion (144).

11. The assembly of any one of the preceding claims, wherein the extension portion (144) extends the entire length between the proximal and distal shoulders (112) and one or more radiopaque markers (146) are positioned at selected locations along the length of the extension portion (144), such as at locations corresponding to the inflow end, mid-section, and outflow end of the prosthetic valve.

12. The assembly of any one of the preceding claims, wherein each shoulder comprises a body (124, 126) and a flared end portion (128, 130).

13. The assembly of claim 12, wherein the flared end portion (128, 130) of the distal shoulder (112) is located at a proximal end portion of the body (124, 126), and the flared end portion (128, 130) of the proximal shoulder (110) is located at a distal end portion of the body (124, 126).

14. The assembly of any one of claims 12 or 13, wherein the flared end portion (128, 130) of each shoulder comprises one or more slots (132), preferably fewer than four slots (132).

15. The assembly of any one of the preceding claims, wherein the prosthetic heart valve (10) comprises a stent or frame (12) and a valvular structure (14) supported by the frame (12) and configured to regulate the flow of blood through the prosthetic valve;
preferably wherein:
the prosthetic valve is adapted to be implanted at the native aortic valve and the frame (12) comprises a nickel-cobalt-chromium alloy;
the valvular structure (14) comprises plurality of leaflets (16) mounted inside of the frame (12);
opposing sides of each leaflet are paired with adjacent leaflets (16) to form commissures (18) of the valvular structure (14);
the commissures (18) are mounted to the frame (12) via reinforcing members (20);
the prosthetic valve includes a sealing member (22) mounted on the outside of the frame (12), wherein the sealing member (22) is configured to help seal the prosthetic valve against surrounding tissue and prevent or minimize perivalvular leakage; and
the reinforcing members (20) and the sealing member (22) are made of a fabric material, such as polyethylene terephthalate fabric;
more preferably wherein the commissures (18) do not extend radially outside of the frame (12).

## Patentansprüche

1. Zuführanordnung, umfassend:
eine ballonexpandierbare Herzklappenprothese (10), die radial zwischen einer gecrimpten Konfiguration und einer expandierten Konfiguration expandierbar ist; und
eine Zuführvorrichtung (100), umfassend:
einen Griff (102),
einen ersten Schaft (104), der sich von dem Griff (102) erstreckt und einen proximalen Endabschnitt, der mit dem Griff (102) gekoppelt ist, und einen distalen Endabschnitt aufweist,
einen zweiten Schaft (106), der sich koaxial über den ersten Schaft (104) erstreckt und einen proximalen Endabschnitt, der mit dem Griff (102) gekoppelt ist, und einen distalen Endabschnitt aufweist;
einen Ballon (118), der über dem ersten Schaft (104) angeordnet ist;
eine proximale Schulter (110) und eine distale Schulter (112), die sich koaxial über den ersten Schaft (104) erstrecken und innerhalb des Ballons (118) angeordnet sind;
einen Verlängerungsabschnitt (144), der sich von der proximalen Schulter (110) erstreckt;
wenigstens eine röntgendichte Markierung (146) auf dem Verlängerungsabschnitt (144); und
wobei die Herzklappenprothese (10) auf dem Ballon (118) in der radial gecrimpten Konfiguration zwischen der proximalen und der distalen Schulter (112) angeordnet ist, derart, dass die wenigstens eine röntgendichte Markierung (146) innerhalb der Herzklappenprothese (10) positioniert ist.

2. Anordnung nach Anspruch 1, wobei die distale Schulter (112) mit dem distalen Endabschnitt des ersten Schafts (104) gekoppelt ist.

3. Anordnung nach einem der vorhergehenden Ansprüche, wobei sich der erste Schaft (104) koaxial durch den Verlängerungsabschnitt (144) erstreckt und nicht direkt an dem Verlängerungsabschnitt (144) befestigt ist.

4. Anordnung nach einem der vorhergehenden Ansprüche, ferner umfassend einen Nasenkonus (114), der mit dem distalen Endabschnitt des ersten Schafts (104) verbunden ist, wobei vorzugsweise ein Führungsdraht sich durch ein zentrales Lumen des ersten Schafts (104) und ein inneres Lumen des Nasenkonus (114) erstrecken kann, so dass die Zuführvorrichtung (100) über den Führungsdraht innerhalb der Gefäße des Patienten vorgeschoben werden kann.

5. Anordnung nach Anspruch 4, wobei der Ballon (118) einen proximalen Endabschnitt, der an einer äußeren Oberfläche des zweiten Schafts (106) befestigt ist, und einen distalen Endabschnitt aufweist, der an einer äußeren Oberfläche des Nasenkonus (114) oder einer äußeren Oberfläche der distalen Schulter (112) befestigt ist.

6. Anordnung nach einem der Ansprüche 4 oder 5, wobei der Nasenkonus (114) und die distale Schulter (112) entlang des ersten Schafts (104) voneinander beabstandet sind.

7. Anordnung nach einem der vorhergehenden Ansprüche, wobei der Verlängerungsabschnitt (144) ein röhrenförmiges Element oder röhrenförmiger Schaft ist, das/der sich über den distalen Endabschnitt des ersten Schafts (104) erstreckt.

8. Anordnung nach Anspruch 7, wobei der Verlängerungsabschnitt (144) einen proximalen Endabschnitt aufweist, der sich in einen distalen Lumenabschnitt der proximalen Schulter (110) erstreckt und darin fixiert befestigt ist.

9. Anordnung nach einem der vorhergehenden Ansprüche, wobei der Verlängerungsabschnitt (144) nicht an dem distalen Endabschnitt des ersten Schafts (104) fixiert ist.

10. Anordnung nach einem der vorhergehenden Ansprüche, wobei die röntgendichte Markierung (146) eine Reihe von röntgendichten Bändern oder ein röntgendichtes Muster ist und entlang der Länge des Verlängerungsabschnitts (144) angeordnet ist.

11. Anordnung nach einem der vorhergehenden Ansprüche, wobei sich der Verlängerungsabschnitt (144) über die gesamte Länge zwischen der proximalen und der distalen Schulter (112) erstreckt und eine oder mehrere röntgendichte Markierungen (146) an ausgewählten Stellen entlang der Länge des Verlängerungsabschnitts (144) positioniert sind, beispielsweise an Stellen, die dem Einströmende, dem Mittelabschnitt und dem Ausströmende der Klappenprothese entsprechen.

12. Anordnung nach einem der vorhergehenden Ansprüche, wobei jede Schulter einen Körper (124, 126) und einen aufgeweiteten Endabschnitt (128, 130) umfasst.

13. Anordnung nach Anspruch 12, wobei sich der aufgeweitete Endabschnitt (128, 130) der distalen Schulter (112) an einem proximalen Endabschnitt des Körpers (124, 126) befindet, und sich der aufgeweitete Endabschnitt (128, 130) der proximalen Schulter (110) an einem distalen Endabschnitt des Körpers (124, 126) befindet.

14. Anordnung nach einem der Ansprüche 12 oder 13, wobei der aufgeweitete Endabschnitt (128, 130) jeder Schulter einen oder mehrere Schlitze (132) umfasst, vorzugsweise weniger als vier Schlitze (132).

15. Anordnung nach einem der vorhergehenden Ansprüche, wobei die Herzklappenprothese (10) einen Stent oder Rahmen (12) und eine Klappenstruktur (14) umfasst, die von dem Rahmen (12) gestützt wird und dazu ausgebildet ist, den Blutfluss durch die Klappenprothese zu regulieren;
vorzugsweise wobei:
die Klappenprothese dazu eingerichtet ist, an der nativen Aortenklappe implantiert zu werden, und der Rahmen (12) eine Nickel-Kobalt-Chrom-Legierung umfasst;
die Klappenstruktur (14) mehrere Segel (16) umfasst, die im Inneren des Rahmens (12) angebracht sind;
gegenüberliegende Seiten jedes Segels mit benachbarten Segeln (16) gepaart sind, um Kommissuren (18) der Klappenstruktur (14) zu bilden;
die Kommissuren (18) über Verstärkungselemente (20) an dem Rahmen (12) angebracht sind;
die Klappenprothese ein Dichtungselement (22) umfasst, das an der Außenseite des Rahmens (12) angebracht ist, wobei das Dichtungselement (22) dazu konfiguriert ist, die Klappenprothese gegen umgebendes Gewebe abzudichten und eine perivalvuläre Leckage zu verhindern oder zu minimieren; und
die Verstärkungselemente (20) und das Dichtungselement (22) aus einem Gewebematerial hergestellt sind, wie beispielsweise Polyethylenterephthalat-Gewebe;
wobei mehr bevorzugt die Kommissuren (18) sich nicht radial außerhalb des Rahmens (12) erstrecken.

## Revendications

1. Ensemble de pose, comprenant :
une valvule cardiaque prothétique (10) déployable par ballonnet, radialement déployable entre une configuration comprimée et une configuration déployée ; et
un appareil de pose (100) comprenant :
une poignée (102) ;
une première tige (104) s'étendant à partir de la poignée (102) et présentant une partie extrémité proximale accouplée à la poignée (102) et une partie extrémité distale ;
une seconde tige (106) s'étendant coaxialement sur la première tige (104) et présentant une partie extrémité proximale accouplée à la poignée (102) et une partie extrémité distale ;
un ballonnet (118) disposé sur la première tige (104) ;
un épaulement proximal (110) et un épaulement distal (112) s'étendant coaxialement sur la première tige (104) et disposés à l'intérieur du ballonnet (118) ;
une partie extension (144) s'étendant à partir de l'épaulement proximal (110) ;
au moins un marqueur radio-opaque (146) sur la partie extension (144) ; et
la valvule cardiaque prothétique (10) étant disposée sur le ballonnet (118) dans la configuration radialement comprimée entre les épaulements proximal et distal (112) de telle sorte que ledit au moins un marqueur radio-opaque (146) est positionné à l'intérieur de la valvule cardiaque prothétique (10).

2. Ensemble selon la revendication 1, l'épaulement distal (112) étant accouplé à la partie extrémité distale de la première tige (104).

3. Ensemble selon l'une quelconque des revendications précédentes, la première tige (104) s'étendant coaxialement à travers la partie extension (144) et n'étant pas fixée fermement, directement à la partie extension (144).

4. Ensemble selon l'une quelconque des revendications précédentes, comprenant en outre une pointe avant (114) reliée à la partie extrémité distale de la première tige (104), un fil-guide pouvant de préférence s'étendre à travers une lumière centrale de la première tige (104) et une lumière interne de la pointe avant (114) de telle sorte que l'appareil de pose (100) peut être avancé sur le fil-guide à l'intérieur du système vasculaire d'un patient.

5. Ensemble selon la revendication 4, le ballonnet (118) présentant une partie extrémité proximale fixée fermement à une surface externe de la seconde tige (106) et une partie extrémité distale fixée fermement à une surface externe de la pointe avant (114) ou à une surface externe de l'épaulement distal (112).

6. Ensemble selon l'une quelconque des revendications 4 ou 5, la pointe avant (114) et l'épaulement distal (112) étant écartés l'un de l'autre le long de la première tige (104).

7. Ensemble selon l'une quelconque des revendications précédentes, la partie extension (144) étant un élément tubulaire ou une tige s'étendant sur la partie extrémité distale de la première tige (104).

8. Ensemble selon la revendication 7, la partie extension (144) présentant une partie extrémité proximale qui s'étend dans une partie lumière distale de l'épaulement proximal (110) et étant fixée fermement à demeure en son sein.

9. Ensemble selon l'une quelconque des revendications précédentes, la partie extension (144) n'étant pas fixée à la partie extrémité distale de la première tige (104).

10. Ensemble selon l'une quelconque des revendications précédentes, le marqueur radio-opaque (146) étant constitué d'une série de bandes radio-opaques ou d'un motif radio-opaque et étant disposé le long de la longueur de la partie extension (144).

11. Ensemble selon l'une quelconque des revendications précédentes, la partie extension (144) s'étendant sur toute la longueur entre les épaulements proximal et distal (112) et un ou plusieurs marqueurs radio-opaques (146) étant positionnés à des emplacements sélectionnés le long de la longueur de la partie extension (144), par exemple à des emplacements correspondant à l'extrémité d'entrée, à la section médiane et à l'extrémité de sortie de la valvule prothétique.

12. Ensemble selon l'une quelconque des revendications précédentes, chaque épaulement comprenant un corps (124, 126) et une partie extrémité évasée (128, 130).

13. Ensemble selon la revendication 12, la partie extrémité évasée (128, 130) de l'épaulement distal (112) étant située au niveau d'une partie extrémité proximale du corps (124, 126) et la partie extrémité évasée (128, 130) de l'épaulement proximal (110) étant située au niveau d'une partie extrémité distale du corps (124, 126).

14. Ensemble selon l'une quelconque des revendications 12 ou 13, la partie extrémité évasée (128, 130) de chaque épaulement comprenant une ou plusieurs fentes (132), de préférence moins de quatre fentes (132).

15. Ensemble selon l'une quelconque des revendications précédentes, la valvule cardiaque prothétique (10) comprenant une endoprothèse ou un cadre (12) et une structure valvulaire (14) supportée par le cadre (12) et conçue pour réguler l'écoulement de sang à travers la valvule prothétique ;
de préférence :
la valvule prothétique étant conçue pour être implantée au niveau de la valvule aortique native et le cadre (12) comprenant un alliage nickel-cobalt-chrome ;
la structure valvulaire (14) comprenant une pluralité de feuillets (16) montés à l'intérieur du cadre (12) ;
des côtés opposés de chaque feuillet étant appariés avec des feuillets (16) adjacents afin de former des commissures (18) de la structure valvulaire (14) ;
les commissures (18) étant montées sur le cadre (12) par l'intermédiaire d'éléments de renforcement (20) ;
la valvule prothétique comportant un élément d'étanchéité (22) monté sur l'extérieur du cadre (12), l'élément d'étanchéité (22) étant conçu pour aider à fermer hermétiquement la valvule prothétique contre un tissu environnant et à empêcher ou à réduire au minimum une fuite périvalvulaire ; et
les éléments de renforcement (20) et l'élément d'étanchéité (22) étant constitués d'un matériau de tissu, tel qu'un tissu de poly(téréphtalate d'éthylène) ;
plus préférablement, les commissures (18) ne s'étendant pas radialement à l'extérieur du cadre (12).
